# EUROPEAN PATENT APPLICATION

(11) **EP 2 517 696 A1**
(43) Date of publication of application: **31.10.2012**
(21) Application number: 12003038.2
(22) Date of filing: 27.04.2012
(51) Int. Cl.: A61K 9/24, A61K 31/13, A61K 9/20

(54) **Extended release memantine tablet**

(30) Priority: 27.04.2011 TR 201104108
(71) Applicant: Ali Raif Ilaç Sanayi ve Ticaret Anonim Sirketi, Istanbul (TR)
(72) Inventor: Bulgur, Abdullah, Istanbul (TR)
(74) Representative: Bulut, Pinar

(57) **Abstract**

The present invention is related to an extended release pharmaceutical composition comprising memantine hydrochloride. The composition is matrix type and comprises polyethylene oxide as polymer controlling the release. It can be formed as two-layer tablet core providing immediate and extended release and immediate release providing coating.

## Description

### Technical Field:

The present invention relates to a two-phase pharmaceutical composition with extended release, which comprises 5 to 30 mg memantine HCl and is administered once a day.

### Prior Art:

It is an active substance known with memantine HCl (3,5 dimethyl-1-adamantamine hydrochloride) chemical name (I). It is used in Alzheimer therapy.

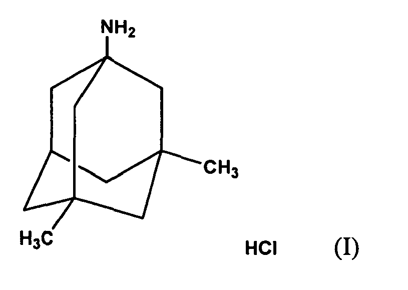

Memantine HCl (memantine hydrochloride) is a water-soluble substance. Although the molecule has two chiral cores, the molecule doesn't have chiral for there isn't plane of symmetry between them. Memantine HCL crystallized with several solvents, is tested with X-Ray powder diffraction and confirmed not to indicate polymorphism. The molecule falls under BCS 1 class with its high dissolution and permeability.

Dosing implemented twice a day in immediate release memantine tablets, causes considerable fluctuations in blood concentrations of active substance, because the absolute bioavailability of memantine is 100%. Therefore, controlled release formulations of the active substance are gaining more importance gradually.

In US2006/0051416 patent, the controlled release is provided with polymer-based matrix system. However, it is not clear that this release can provide the blood level required for therapeutic effect with the blood concentrations obtained in the early hours.

US 5,382,601 belongs to memantine HCl solid dosage form presenting two-phase release profile, a part of which releases immediately and the other part releases more slowly. This matrix tablet comprises water-soluble and water-insoluble casein and its salts. Adverse effects regarding casein and abnormal stability problems at different pHs are known.

WO 2006/138227 provides the required two-phase release with immediate release and controlled release memantine pellet mixture. As is known, pellet production is an intensive, long-term and high-loss procedure.

### Description of the Invention:

The present invention is related with formulation of extended release tablets comprising 5 to 30 mg memantine HCl bioequivalent to 5, 10, 15 and 20 mg immediate release memantine tablets present on the market and implemented once a day.

According to the present invention, extended release polymer is obtained with memantine extended release tablet form that can be easily produced by compressing the controlled part as two layer tablet with immediate release part or compressing it alone and coating by coating formula with active content, and can be implemented in normal production methods. The composition is a matrix type formulation and includes a polymer controlling the release. Polyethylene oxide polymer is chosen as polymer. The molecular weight of the polyethylene oxide molecule of the polyethylene oxide polymer used is 7,000,000 g/mole and Brookfield viscosity of its 1% m/v aqueous solution at 25°C is 7500-10000 cps (Colorcon - Sentry Polyox WSR 303 - Leo - NF - Dow).

The extended release tablet developed, realizes the extended release between 20%-40% in the first hour in order to provide the adequate effective blood level and two-phase pellets to achieve Cₘₐₓ value. Then in order to keep the memantine plasma concentration effective in the long run, it provides 8-hour and longer term release of the remaining memantine amount. In dissolution test, 20% to 40% of memantine HCl is released in the first hour at 0.1N HCl medium. The other part of the composition provides 5% to 12% release per hour in pH 6.8 phosphate buffer.

The tablet developed is composed of two parts: one part providing immediate release and the other part providing extended release. 65-85% of memantine HCl content of the tablet is present at extended release providing part and 15-35% is present at immediate release providing part. The two seperate parts can be compressed as two layer tablet. In the another embodiment of the invention, the part providing extended release forms the tablet core and the part providing immediate release is coated on the tablet core.

In the extended release providing part, there is found calcium phosphate dibasic as buffer agent, talk and magnesium stearate as lubricant and colloidal silicon dioxide (Aerosil 200) as glidant. In the two layer tablet composition, microcrystalline cellulose as diluent and binder, croscarmellose sodium as disintegrant, magnesium stearate as lubricant and colloidal silicon as glidant are used in the part providing immediate release. In the formulation, two types of microcrystalline cellulose known with Avicel PH101 and Avicel PH102 trade name, are used together.

In the coated tablet formulation, appropriate coating polymers can be used in immediate release providing coating part. The preferred polymer is hydroxypropylmethylcellulose (HPMC) and in more preferred condition, hydroxypropylmethylcellulose 3 cps known with Methocel E3-LV trade name is used. The coating layer comprises polyvinylpyrrolidone (Povidon K-30) and polyethylene glycol apart from HPMC. Polyethylene glycol with 5600-6600 g/mole molecular weight, is used as polyethylene glycol (Clariant Polyglycol 6000 P). It is known as PEG 6000, shortly.

The examples related to subject of the invention are given below, but the subject of the invention is not restricted with the examples given.

### Example1 :

The formulations of immediate release and extended release parts are given in Table 1.1 and 1.2.

**Table 1.1 Immediate release part**

| | | |
|---|---|---|
| 1 | Memantine HCl | 1.25mg |
| 2 | Avicel PH 101 | 3mg |
| 3 | Aerosil 200 | 0.5mg |
| 4 | Avicel PH 102 | 72.5mg |
| 5 | Croscarmellose | 2mg |
| 6 | Magnesium Stearate | 0.75mg |
| | TOTAL | 80mg |

Preparation: 1,2 and 3 are mixed and sieved, and then mixed again. 4 and 5 are added after sieving. 6 is added after sieving by 30 mesh sieve. The mixture is stirred for 5 minutes.

**Table 1.2. Extended release part**

| | | |
|---|---|---|
| 1 | Memantine HCl | 5.75mg |
| 2 | Polyethyleneoxide | 300mg |
| 3 | Calcium Phosphate Dibasic | 77.25mg |
| 4 | Aerosil 200 | 4mg |
| 5 | Talk | 9mg |
| 6 | Magnesium Stearate | 4mg |
| | TOTAL | 400mg |

Preparation : 1, 2, 3, 4 are sieved and mixed. 5 and 6 are sieved with 30 mesh sieve and then added. The mixture is stirred for 5 minutes.

The two laters are compressed at minimum 10 kp hardness by double-fed tablet machine. The tablets are tested by basket type dissolution device at 100 rpm, in 500 ml 0.1 N HCl medium for an hour and then they are tested at 500 ml pH 6.8 phosphate buffer for 9 hours again, to be 10 hours in total.

**Table 1.3. Dissolution profile**

| 0.1 N HCl | | | | pH 6.8 Phosphate buffer | | | | | | | | |
|---|---|---|---|---|---|---|---|---|---|---|---|---|
| Time (hours) | ¼ | ½ | 1 | 1 | 2 | 3 | 4 | 5 | 6 | 7 | 8 | 9 |
| Dissolution (%) | 10 | 28 | 38 | 46 | 53 | 60 | 67 | 74 | 80 | 86 | 92 | 100 |

### Example 2:

Immediate and extended release parts and tablets are obtained as in Example 1 and tested with the method given in Example 1.

**Table 2.1. Immediate release part**

| | | |
|---|---|---|
| 1 | Memantine HCl | 5mg |
| 2 | Avicel PH 101 | 3mg |
| 3 | Aerosil 200 | 0.5mg |
| 4 | Avicel PH 102 | 68.75mg |
| 5 | Croscarmellose | 2mg |
| 6 | Mg Stearate | 0.75mg |
| | TOPLAM | 80mg |

**Tablo 2.2. Extended release part**

| | | |
|---|---|---|
| 1 | Memantine HCl | 23mg |
| 2 | Polyethyleneoxide | 300mg |
| 3 | Calcium Phosphate Dibasic | 60mg |
| 4 | Aerosil 200 | 4mg |
| 5 | Talk | 9mg |
| 6 | Magnesium Stearate | 4mg |
| | TOTAL | 400mg |

**Table 2.3. Dissolution Profile**

| 0.1 N HCl | | | | pH 6.8 Phosphate buffer | | | | | | | | |
|---|---|---|---|---|---|---|---|---|---|---|---|---|
| Time (hour) | ¼ | ½ | 1 | 1 | 2 | 3 | 4 | 5 | 6 | 7 | 8 | 9 |
| Dissolution (%) | 9 | 30 | 39 | 48 | 56 | 63 | 70 | 77 | 84 | 90 | 97 | 101 |

### Example 3:

Immediate and extended release parts and tablets are obtained as in Example 1 and tested with the method given in Example 1.

**Table 3.1. Immediate release part**

| | | |
|---|---|---|
| 1 | Memantine HCl | 5mg |
| 2 | Avicel PH 101 | 3mg |
| 3 | Aerosil 200 | 0.5mg |
| 4 | Avicel PH 102 | 68.75mg |
| 5 | Croscarmellose | 2mg |
| 6 | Mg Stearate | 0.75mg |
| | TOTAL | 80mg |

**Table 3.2. Extended release part**

| | | |
|---|---|---|
| 1 | Memantine HCl | 23mg |
| 2 | Polyethyleneoxide | 330mg |
| 3 | Calcium phosphate dibasic | 30mg |
| 4 | Aerosil 200 | 4mg |
| 5 | Talk | 9mg |
| 6 | Magnesium Stearate | 4mg |
| | TOTAL | 400mg |

**Table 3.3. Dissolution profile**

| 0.1 N HCl | | | | pH 6.8 Phosphate buffer | | | | | | | | |
|---|---|---|---|---|---|---|---|---|---|---|---|---|
| Time (hour) | ¼ | ½ | 1 | 1 | 2 | 3 | 4 | 5 | 6 | 7 | 9 | 12 |
| Dissolution (%) | 10 | 24 | 31 | 41 | 51 | 58 | 65 | 72 | 79 | 84 | 91 | 99 |

### Example 4:

The tablet core providing extended release, is covered with a coating comprising active substance as immediate release providing part.

**Table 4.1. Tablet core and coating formulation**

| Extended release part | | |
|---|---|---|
| 1 | Memantine HCl | 23mg |
| 2 | Polyethylenoxide | 330mg |
| 3 | Calcium Phosphate Dibasic | 30mg |
| 4 | Aerosil 200 | 4mg |
| 5 | Talk | 9mg |
| 6 | Magnesium Stearate | 4mg |
| | TOTAL | 400mg |

| Coating with Active Substance | | |
|---|---|---|
| 7 | Memantine HCl | 5mg |
| 8 | Hydroxypropylmethylcellulose 3 cps | 6.4mg |
| 9 | Povidone K-30 | 0.5mg |
| 10 | Polyethylene Glicol | 0.1mg |
| | TOTAL | 412mg |

Preparation: 1,2,3,4 are sieved and mixed. 5 and 6 are sieved with 30 mesh sieve and added. The mixture is mixed for 5 minutes.

400 mg tablet is compressed in round convex moulds at 10-12 kp hardness.

Coating solution is prepared by dissolving 7, 8, 9, 10 in water. The tablets at 50°C inlet air temperature are coated until they become 211.5 mg.

Tablets are tested by basket type dissolution device at 100 rpm, in 500 ml 0.1 N HCl medium for an hour and then they are tested at 500 ml pH 6.8 phosphate buffer during 12 hours.

Dissolution profile obtained is given in Table 4.2.

**Table 4.2. Dissolution test**

| 0.1 N HCl | | | | pH 6.8 Phosphate buffer | | | | | | | | |
|---|---|---|---|---|---|---|---|---|---|---|---|---|
| Time (hour) | ¼ | ½ | 1 | 1 | 2 | 3 | 4 | 5 | 6 | 7 | 9 | 12 |
| Dissolution (%) | 12 | 17 | 26 | 33 | 41 | 49 | 56 | 63 | 71 | 77 | 84 | 91 |

### Example 5:

Tablets are obtained as in Example 4 and tested with the method given in Example 4.

**Table 5.1. Tablet core and coating formulation**

| Extended release part | |
|---|---|
| Memantine HCl | 5.75mg |
| Polyethyleneoxide | 300mg |
| Calcium phosphate dibasic | 77.25mg |
| Aerosil 200 | 4mg |
| Talk | 9mg |
| Magnesium Stearate | 4mg |
| TOTAL | 400mg |

| Coating with Active Coating | |
|---|---|
| Memantine HCl | 1.25mg |
| Hydroxypropylmethylcellulose 3 cps | 8.15mg |
| Povidone K-30 | 0.5mg |
| Polyethylene Glycol | 0.1 mg |
| TOTAL | 410mg |

**Table 5.2 Dissolution test**

| 0.1 N HCl | | | | pH 6.8 Phosphate buffer | | | | | | | | |
|---|---|---|---|---|---|---|---|---|---|---|---|---|
| Time (hour) | ¼ | ½ | 1 | 1 | 2 | 3 | 4 | 5 | 6 | 7 | 9 | 12 |
| Dissolution (%) | 10 | 24 | 35 | 45 | 52 | 61 | 66 | 74 | 81 | 87 | 94 | 102 |

### Bioequivalence study:

In the study, extended release Memantine tablet (Formula 4 = Example 4) is compared with one conventional immediate release tablet Ebixa 20 mg (Batch No. 946005) on the market.

The study is carried out on 12 healthy volunteers as clear label pilot bioequivalence study. Single dose Memantine 28 mg SR (Formula 4) is given to one group and single dose Ebixa 20 mg tablet is given to the other group.

For memantine serum concentration assay, 1 hour before Memantine 28 mg SR implementation and at 1,2,4,5,6,8,9,10,12,24,36,48,72 hours after the implementation, the blood sample is taken. 1 hour before and after the reference product Ebixa 20 mg, blood sample is taken at 1,2,4,6,7,8,9,10,12,24,36,48,72 hours.

At every turn, 7 ml blood is taken from the volunteers. Average AUCₒ₋ₜ, AUC_{o-∞}, Cₘₐₓ and Cₘᵢₙ are calculated from the serum measurements of the volunteers. The results are given in Table 6.1.

**Table 6.1: Bioequivalence study results**

| IMPLEMENTATION | AUC₀₋₇₂ | AUC_{o-∞} | Cₘₐₓ | Tₘₐₓ | Cₘᵢₙ |
|---|---|---|---|---|---|
| Ebixa 20 mg T | 1106.25 | 1738 | 24.69 | 5 hours | 9.24 |
| Formulation 4 | 1131.24 | 1663 | 22.96 | 12 hours | 9.34 |

**Table 6.2: Bioequivalence test - Memantine 28 mg SR Tablet (Formula 4)**

| Time (saat) | Plasma Concentration ng/ml | | Parameter | Value | Unit |
|---|---|---|---|---|---|
| | | | Intersection | 32.92819362 | ng/ml |
| 1 | 1.86 | | Slope | 0,017554452 | h-1 |
| 2 | 4.68 | | t1/2 | 39.47716529 | h |
| 4 | 10.68 | | AUC 0-tn | 1131.24 | h*ng/ml |
| 5 | 15.23 | | AUC 0-inf | 1663.298764 | h*ng/ml |
| 6 | 16.05 | | MRT | 63.43925908 | h |
| 8 | 17.23 | | Cmax | 22,96 | ng/ml |
| 9 | 18.79 | | Tmax | 12 | h |
| 10 | 20.23 | | tn | 72 | h |
| 12 | 22.96 | | Ctn | 9.34 | ng/ml |
| 24 | 21.54 | | | | |
| 36 | 17.75 | | | | |
| 48 | 13.97 | | | | |
| 72 | 9.34 | | | | |

**Table 6.3: Bioequivalence test data - Ebixa 20 mg Tablet**

| Time (hour) | Plasma concentration ng/ml | | Parameter | Value | Unit |
|---|---|---|---|---|---|
| 0,00 | | | Intersection | 26.39022035 | ng/ml |
| 1 | 3.8 | | Slope | 0,014614566 | h-1 |
| 2 | 8.23 | | t1/2 | 47.41844486 | h |
| 4 | 18.57 | | AUC 0-tn | 1106.25 | h*ng/ml |
| 5 | 24.69 | | AUC 0-inf | 1738.495931 | h*ng/ml |
| 6 | 24.05 | | MRT | 70.89883823 | h |
| 8 | 23.11 | | Cmax | 24.69 | ng/ml |
| 9 | 22.65 | | Tmax | 5 | h |
| 10 | 22.54 | | tn | 72 | h |
| 12 | 22.12 | | Ctn | 9.24 | ng/ml |
| 24 | 18.56 | | | | |
| 36 | 15.72 | | | | |
| 48 | 12.96 | | | | |
| 72 | 9.24 | | | | |

### Description of the Figures

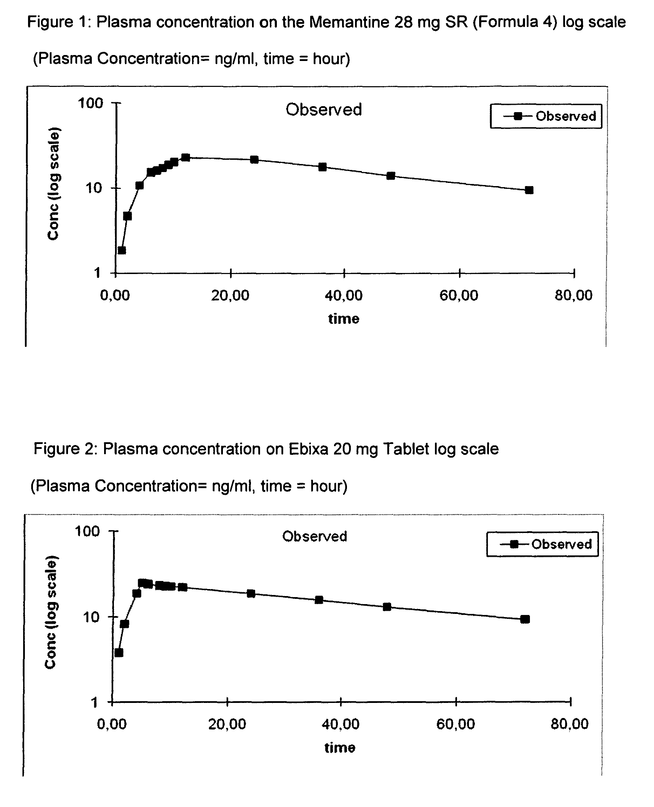

Figure 1: Plasma concentration of Memantine 28 mg SR (Formula 4) on the log scale
Figure 2: Plasma concentration of Ebixa 20 mg Tablet on the log scale

## Claims

1. Extended release tablet comprising memantine hydrochloride, **characterized in that** it is two phase as immediate release part and extended release part, and extended release providing part comprises polyethylene oxide as gel forming polymer.

2. Extended release tablet according to Claim 1, **characterized in that** the ratio of polyethylene oxide present in the extended release part is 70-85 % of the extended release part by weight.

3. Extended release tablet according to Claim 1 and 2 **characterised in that** the molecular weight of polyethylene oxide polymer is 7,000,000 g/mole and Brookfield viscosity value at 25°C of 1% m/v aqueous solution is 7500-10000 cps.

4. Extended release tablet according to Claim 1 **characterised in that** one tablet comprises 5 to 30 mg memantine hydrochloride.

5. According to Claim 1, 65-85 % of memantine hydrochloride content is found in extended release part and 15-35 % of it is found in immediate release part.

6. Extended release tablet according to Claim 1, extended release part is the tablet core and immediate release part is coating layer.

7. Extended release tablet according to Claim 6, **characterized in that**:
a) Extended release providing core comprises 5 to 25 mg memantine hydrochloride, polyethylene oxide, calcium phosphate dibasic, colloidal silicon dioxide, talk and magnesium stearate,
b) Immediate release providing coating comprises 1 to 5 mg memantine hydrochloride, hydroxypropyl methylcellulose, polyvinylpyrolidone and polyethylene glycol.

8. According to Claim 1, extended release tablet is a two-layer tablet.

9. Two-layered extended release tablet according to Claim 8, **characterized in that**;
a) Extended release providing part comprises 5 to 25 mg memenatine hydrochloride, polyethylene oxide, calcium phosphate dibasic, colloidal silicon dioxide, talk and magnesium stearate,
b) Immediate release providing part comprises 1 to 5 mg memenatine hydrochloride, microcrystalline cellulose, croscarmellose sodium, colloidal silicon dioxide and magnesium stearate.

10. Extended release tablet according to the previous claims, **characterized in that** 20-40 % of memantine hydrochloride amount is released by rotating basket type, dissolution rate assay device, at 100 rpm in 500 ml 0.1 N hydrochloric acid medium in an hour; later then, memantine hydrochloride in the rate of 5-12 % of memantine hydrochloride amount is released in 500 ml pH 6.8 phosphate buffer per hour.
